(19) **Europäisches Patentamt / European Patent Office / Office européen des brevets**

(11) **EP 1 397 064 B1**

(12) **EUROPEAN PATENT SPECIFICATION**

(45) Date of publication and mention
of the grant of the patent:
**17.06.2009 Bulletin 2009/25**

(51) Int Cl.:
***A47K 7/02*** *(2006.01)*

(21) Application number: **02723799.9**

(22) Date of filing: **10.04.2002**

(86) International application number:
**PCT/US2002/011013**

(87) International publication number:
**WO 2002/100231 (19.12.2002 Gazette 2002/51)**

(54) **COATED OIL ABSORBING WIPES**

BESCHICHTETE ÖL ABSORBIERENDE WISCHTÜCHER

LINGETTES ENDUITES SERVANT À ABSORBER D'HUILE

(84) Designated Contracting States:
**AT BE CH CY DE DK ES FI FR GB GR IE IT LI LU MC NL PT SE TR**

(30) Priority: **07.06.2001 US 876704**

(43) Date of publication of application:
**17.03.2004 Bulletin 2004/12**

(73) Proprietor: **3M Innovative Properties Company
St. Paul MN 55133-3427 (US)**

(72) Inventors:
• **MROZINSKI, James, S.
Saint Paul, MN 55133-3427 (US)**

• **SETH, Jayshree
Saint Paul, MN 55133-3427 (US)**
• **THORSON, James, E.
Saint Paul, MN 55133-3427 (US)**

(74) Representative: **Vossius & Partner
Siebertstrasse 4
81675 München (DE)**

(56) References cited:
**WO-A-01/85001        WO-A-99/29220
US-A- 4 279 890       US-A- 4 643 939
US-A- 5 744 149       US-B1- 6 214 362**

**Description**

This invention relates to oil absorbent skin wipe products. The invention particularly relates to oil absorbent skin wiping products with additional functional layers.

A significant amount of oil continuously oozes out of the skin of the face, particularly the nose, cheek, forehead and middle forehead. To maintain cleanliness and to improve the spreadability of cosmetics it is important to remove any excess oil or sebum. Soap and water work to some extent but there are always times when one is not able to wash. Dry methods of removing these facial oils include the use of thin oil absorbent wipe materials. Oil absorbing wipes for removing facial oil have also been described in the art. These wipes generally must be thin, conformable and non-abrasive, considerations not relevant to industrial oil absorbent materials.

Conventional paper type wipes have been used to remove facial oil. For example, natural or synthetic papers using vegetable fibers, synthetic pulp or kenaf have been used. These oil absorbent papers however are often irritating to the skin due to the hard and stiff nature of the fibers. To improve their smoothness, these papers have been calendered and/or coated with powders such as calcium carbonate and sizing agents. Calendering however is not necessarily permanent and surface fibers can reform into a rough surface unless substantial amounts of binder or sizing agents are used, which decrease oil absorption. Paper wipes are also poor indicators as to their effectiveness as papers generally do not significantly change appearance when they have absorbed oil or sebum.

Improvements to oil absorbing papers are described in Japanese Kokai No. 4-45591 which teaches adhering porous spherical beads onto the surface of an oil absorbing paper so as to solve the problems caused by calendering or coating of paper with powders such as calcium carbonate powders. These beads also are used to allegedly increase the capacity of the papers to absorb sebum. Japanese Unexamined Patent Publication (Kokai) No. 6-319664 discloses a high-density oil absorbing paper produced by mixing (a) a pulp material containing vegetable fibers, as the main component with (b) an inorganic filler, followed by paper-making to form a paper with a basis weight of 0.7 $g/cm^2$ or more. However, the oil absorbing papers disclosed in these patent publications still have a limited capacity to absorb oil or sebum and little indicating function as there is little change in opacity or color in the paper when oil is absorbed. Difficultly in confirming oil means that users of the oil clearing paper can not evaluate if or how much sebum is removed from the users' faces when using the oil absorbing paper such that makeup can be applied with confidence.

An oil absorbing paper for sebum is also disclosed in Japanese Examined Patent Publication (Kokoku) No. 56-8606, or U.S. Patent No. 4,643,939, which describes a cosmetic oil absorbing paper produced by mixing hemp fibers with 10 to 70% by weight of polyolefin resin fibers and making a paper with a basis weight of from 12 to 50 $g/cm^2$. This paper will allegedly clear upon absorption of oil but still requires conventional papermaking techniques and would be rough to the touch. Japanese Unexamined Utility Model Publication (Kokai) No. 5-18392, discloses an oil absorbing synthetic paper comprising an oil absorbing paper with a smooth surface coating of inorganic or organic powder material such as clay particles, silica fine-particles, and powdered fibers. These oil-absorbing papers allegedly have some oil indicating effect by clarifying the paper upon oil absorption thus confirming oil absorption. However, the powder coating lowers the oil absorption capacity for these papers and it is still difficult to attain a clear change in the appearance of this type of oil clearing paper after oil absorption.

Oil-absorbing webs produced by using thermoplastic fibrous material in place of cellulosic fibrous papers are known. Further, Japanese Unexamined Patent Publication (Kokai) No. 9-335451 (WO99/29220) discloses an oil wipe made of a porous thermoplastic film. This oil absorbing wipe film has higher oil absorption capacity than the oil absorbing papers and is also superior in confirming removal of oil following wiping as compared to oil absorbing papers. It is believed that the reason for this good oil removal indicating functionality is that these porous thermoplastic films exhibit low light transmittance before oil absorption because of irregular reflection of light, but the light transmittance increases substantially after the micro-pores of the film are filled with oils producing a large change in the film's opacity or light transmittance, and therefore appearance. This change in opacity clearly confirms to the user the removal of oil or sebum from his or her skin. U.S. Patent No. 4,532,937 to Miller describes analytical film for collecting sebum as it is secreted from the sebaceous glands of a subject comprising an open-celled, microporous and hydrophobic polymeric film, and a fibrous material having coated on one major surface a layer of synthetic, pressure-sensitive adhesive consisting essentially of high molecular weight components. The Miller patent describes its material as having pores of such a size and distribution that the film is opaque or opalescent when the pores are empty or filled with air but can become translucent or transparent upon absorption of a liquid such as sebum. However, the very small pores described for this film or material (less than 0.1 microns) do not provide a material best suited for use in cosmetic applications due to the slow oil absorption rates.

U.S. Patent No. 6,214,362 discloses a specific cosmetic pad for either absorbing low tension substances from the skin or applying and smoothing new makeup.

It is an object of the invention to form an oil absorbing wipe having a clear oil indicating function, such as described in WO99/29220, which can also deliver other agents or treatments to the skin following oil removal which product is easy to directly manufacture. Further, it is desirable that these additional agents are clearly visible on the oil absorbing wipe so as to inform the user as to which side of the wipe to use to remove facial oil and which side of the wipe to use to apply or use the additional skin treatment.

The invention is directed at an oil absorbing wipe material for wiping a user's skin. The wipe comprises an oil absorbing porous substrate having two faces and having a transparency of less than 65 percent which porous substrate changes transparency upon absorption of oil. The porous substrate has a non-tacky flexible coating on at least a portion of at least one face. The coating comprises a film forming polymer with at least one additional additive which coating is visible on the coated face of the porous substrate and which coating does not penetrate to the opposite face of the porous substrate. The film forming polymer coating penetrates from 10 to 90 percent of the thickness of the oil absorbing porous substrate. Generally, the additional ingredient is an active or skin codifying agent.

The invention is further directed to a method for forming a flexible coating on an oil absorbing wipe material suitable for wiping a users skin comprising providing an oil absorbing porous substrate having two faces wherein the substrate has a transparency of less than 65 percent which porous substrate changes transparency upon absorption of oil, coating the porous substrate on at least a portion of at least one face with a coating solution comprising at least a film forming polymer, a particulate filler and an evaporative solvent with at least one additional additive, the coating solution having a viscosity of from 2000 to 100,000 cps and a percent solids of 60 to 80 percent wherein the coating is visible on the coated face of the porous substrate and which coating does not penetrate to the opposite face of the porous substrate.

Fig. 1 is a schematic diagram of an apparatus suitable for use in forming the wipes of the invention.

Fig. 2 is a perspective view of a dispensable package of oil absorbing wipes.

Fig. 3 is a perspective view of a dispensable package of oil absorbing wipes according to a second embodiment.

Fig. 4 is a perspective view of a dispensable package of oil absorbing wipes according to a third embodiment.

Fig. 5 is a perspective view of a dispensable package of oil absorbing wipes according to a fourth embodiment.

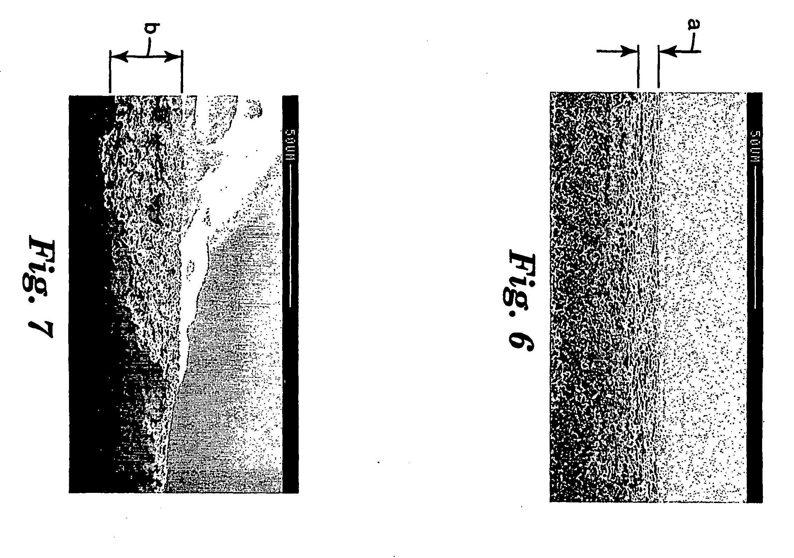

Fig. 6 is a side view photomicrograph of counter example C7 showing the penetration of a brittle film forming coating into an oil absorbing wipe.

Fig. 7 is a side view photomicrograph of example 2 showing the penetration of a flexible film forming coating into an oil absorbing wipe.

Detailed Description

The oil absorbent wipe material of the present invention may be a porous filmlike thermoplastic material which in a first preferred embodiment is generally a porous stretched or oriented film made of a thermoplastic material or alternatively in a second preferred embodiment a consolidated porous nonwoven fiber web which is filmlike. Filmlike as used herein is defined as thermoplastic films or consolidated nonwovens of fibers. In a less preferred embodiment, conventional paperlike oil absorbent wipes can be coated with the film forming polymer layer described in the invention. The wipe will be coated on at least a portion of one face or side with a film forming polymer generally having an additional useful ingredient within the film forming polymer layer.

The porosity of the interstitial volume per unit area of the porous film material of the oil absorbent wipe of the first preferred embodiment is preferably in the range of 0.0001-0.005 cm$^3$ as calculated by the equation:

$$\text{Interstitial volume per unit area} = [\text{film thickness (cm)} \times 1 \text{ (cm)} \times 1 \text{ (cm)} \times \text{void content (\%)}] / 100 \text{ (where the void content is the percentage of voids in the porous film).}$$

The "void content" is more specifically defined as the percentage of an amount of filling material, when all of the voids of the porous film are filled with a material of the same composition as the film, with respect to a film with no corresponding voids. The void content of the porous film is preferably in the range of 5-50% and the thickness is preferably in the range of 5-200μm.

The porous stretched film may be produced by various different methods using a thermoplastic material as the starting substance. In one preferred method, the film is produced by adding a filler to a transparent crystalline thermoplastic resin, forming a film using conventional methods such as blown extrusion or casting, and then stretching the film to create fine voids therein. A porous stretched thermoplastic film obtained in this manner has a large percentage of voids constituting the volume of the wipe compared to conventional paper oil cleaning wipes, and has excellent absorption of skin oils per unit area. Also, since the thermoplastic film has a structure with a uniform distribution of many fine voids, prior to wiping of skin oils from the skin surface it appears non-transparent due to light dispersion by the pore structures. However, after oil absorption the oils fill the voids or pores thus either preventing or reducing the degree of light dispersion. This together with the original opaque or transparent nature of the thermoplastic forming the film allows the oil absorbing effect to be clearly assessed by a change in transparency or opacity.

Examples of transparent crystalline thermoplastic resins which can be used as the film forming material for production of the porous unstretched thermoplastic film of the invention include, but are not limited to, polyethylene, polypropylene, polybutylene, poly-4-methylpentene and ethylene-propylene block copolymer.

Examples of preferred nonparticulate fillers that can be used in combination with the aforementioned thermoplastic resins to provide the fine voids include, but are not limited to, mineral oils, petroleum jelly, low molecular weight polyethylene, soft Carbowax™ and mixtures thereof. These nonparticulate fillers are preferred as they exhibit transparency upon absorption of oil. Mineral oils are preferred among these fillers because of their relatively low cost. However, additionally conventional particulate based fillers can also be used to form the porous film, such as talc, calcium carbonate, titanium dioxide, barium sulfate, etc.

The aforementioned fillers can be varied within a wide range within the starting thermoplastic resin used for production of the film. The amount of filler used is preferably in the range of 20-60% by weight, and more preferably 25-40% by weight of the starting thermoplastic material. If the amount of filler added to the starting material is under 20% by weight, the void content of the film resulting after stretching is reduced, thus lowering the amount of oil absorption, while if it is above 60% by weight it becomes more difficult to produce flexible coherent films.

Other additives may also be added as necessary in addition to the thermoplastic resin and filler in the production of the porous stretched thermoplastic film. For example, organic acids such as carboxylic acid, sulfonic acid and phosphonic acid, and organic alcohols. As additional suitable additives there may also be mentioned, for example, inorganic and organic pigment, aromatic agents, surfactants, antistatic agents, nucleating agents and the like.

The main starting materials and optional additives are melted and/or combined to form a film, producing a filler-containing thermoplastic film. The melting and mixing step(s) and the subsequent film forming step may be carried out according to known methods. An example of a suitable melt mixing method is kneading with a kneader, and examples of suitable film forming methods are the blown film method and the casting method. The blown film method, for example, can give tube-shaped films by melt mixing the main starting material, etc. and then blowing it up from a circular die. The casting method can give films by melt mixing the main starting material, etc. and then extruding it from a die onto a smooth or patterned chilled roll (cold roll). In a modified form of this casting method, the nonparticulate additives and/or fillers may be removed by washing off or extracting with a suitable solvent after extrusion of the melted mixture onto the chilled roll.

The formed thermoplastic film is then stretched to provide it with fine voids. As with the film forming, the stretching may also be carried out according to known methods, such as uniaxial stretching or biaxial stretching. For example, in the case of biaxial stretching, the stretching in the lengthwise direction may be accomplished by varying the speed of the driving roll, and the stretching in the widthwise direction may be accomplished by mechanical pulling in the widthwise direction while holding both sides of the film with clips or clamps.

The conditions for the film stretching are not particularly restricted, but the stretching is preferably carried out so as to give a void content in the range of 5-50% and a stretched film thickness in the range of 5-200 $\mu$m. If the void content upon stretching of the film is under 5% the amount of oil absorption will be reduced, while if it is over 50% the amount of oil absorption will be too great, making it difficult to clearly assess the oil absorbing effect. Also, if the film thickness is under 5 $\mu$m the amount of oil absorption capacity will be too low and the film will tend to adhere to the face making it more difficult to handle, while if it is over 200 $\mu$m the amount of oil absorption capacity will be too great and the film may-feel stiff and harsh against the user's skin.

The stretching ratio for the thermoplastic film is usually preferred to be in the range of 1.5 to 3.0. If the stretching ratio is under 1.5 it becomes difficult to achieve a sufficient void content for oil absorption, while if it is over 3.0 the void content could become too large, causing too much oil absorption.

The average size of the voids formed by stretching of the film is usually preferred to be in the range of 0.2 to 5 $\mu$m If the void size is under 0.2 $\mu$m it becomes impossible to rapidly absorb enough skin oil to create a clear change in transparency, while if it is over 5 $\mu$m the amount of oil absorption needed to permit a visible change in transparency may be too great.

As mentioned above, the interstitial volume per unit area of the porous stretched thermoplastic film obtained by the stretching process described earlier is preferably in the range of 0.0001-0.005 cm$^3$ and more preferably in the range of 0.0002-0.001 cm$^3$, as calculated by the equation defined above. If the interstitial volume of the film is under 0.001 cm$^3$ it becomes difficult for the user to hold the oil cleaning wipe, while if it is over 0.005 cm$^3$ the amount of oil absorption is too great, and it becomes difficult to clearly assess the oil absorbing effect.

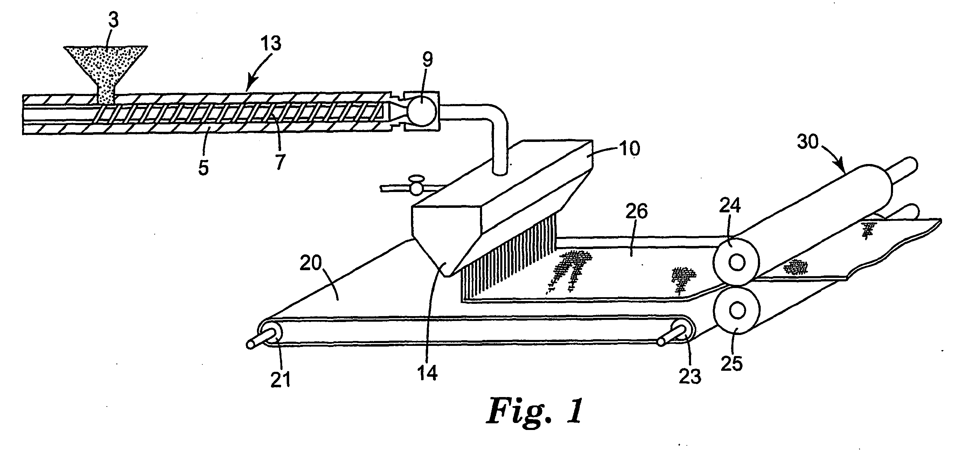

The second embodiment of a film-lihe porous wipe is a consolidated nonwoven web preferably formed of thermoplastic microfibers. A representative apparatus useful for preparing such a web or wipe product is shown schematically in Fig. 1. Part of the apparatus for forming blown fibers is described in Wente Van A., "Superfine Thermoplastic Fibers" in Industrial Engineering Chemistry, Vol. 48, p. 1342 et seq. (1956), or in Report No. 4364 of the Naval Research Laboratories, published May 25, 1954, entitled "Manufacture of Superfine Organic Fibers", by Wente, V.A.; Boone, C. D.; and Fluharty, E.L. Modifications to this basic design are discussed in U.S. Patent Nos. 4,818,463; 3,825,379; 4,907,174 and 4,986,743. This portion of the illustrated apparatus comprises a die 10, which has a set of aligned side-by-side parallel die orifices 14. The die orifices 14 open from a central die cavity. Typically, the diameter of the orifices will be on the order of from about 250 microns to about 500 microns. From about 2 to about 20 such orifices will be provided per linear centimeter of die face. Typically, the length of the orifices will be from about 1 mm to about 5 mm. The polymer is introduced to the die orifices 14 and the central die cavity from a melt extruder 13 having a resin hopper 3, a barrel 5, and a screw 7 inside the barrel 5. The molten polyolefin resin exits from the extruder barrel 5 into a gear melt pump 9 which permits improved control over the flow of the molten polymer through the downstream components of the apparatus. Upon exiting from the pump 9, the molten resin flows into a die 10 containing the die cavity through which liquefied fiber-forming material is advanced. The fiber forming thermoplastic polymer is extruded from the die orifices 14 into an attenuating airstream of heated air. This attenuating airstream is maintained at high velocities and exits from orifices or slots on either side of the set of die orifices 14. The high-velocity air is supplied to slots from two peripheral cavities. The heated air is generally about the temperature of the polymer melt or higher (e.g., 20 to 30°C above the melt temperature).

The fibers exiting from the die orifices are attenuated by the high velocity heated air from slots and are collected on collector 20, such as a belt, at a distance $\underline{a}$ from the die. The distance $\underline{a}$ is generally from 10 to 25 cm with different preferred regions for different polymers depending on the crystalline behavior of the polymer, how rapidly it is quenched to a totally non-tacky condition or other process conditions. The collector can be a flat screen, a drum, a cylinder or a finely perforated collector 20 as shown in Fig. 1. Cylinders 21 and 23 drive the belt 20. A gas-withdrawal device can be located behind perforated collectors to facilitate collection of the fibers, on the screen or other perforated collector surface, as a web 26. From the collector 20, the web 26 is taken to a calender 30 where the web is consolidated under pressure, preferably from 500 to 1600 Newtons per lineal centimeter. This consolidation is advantageously carried out by calendering in the nip between two generally smooth rolls 24 and 25 (e.g., they contact each other over about 90 percent of their surface area or greater, preferably 99 percent or greater), having a Shore A durometer hardness of about 50 or more, although one roll preferably has a Shore A durometer hardness of less than about 95. The consolidated web can then be collected and subsequently converted into individual wipes.

The webs are formed of fiber-forming thermoplastic materials, which materials include, for example, polyolefins, such as polyethylene, polypropylene or polybutylene; polyesters, such as polyethylene terephthalate or polybutylene terephthalate; polyurethanes or polyamides such as nylon 6 or nylon 66. The microfibers have an average diameter of less than 10 micrometers, preferably with an average diameter of 7 micrometers or less. Smaller average fiber diameters may be obtained with smaller diameter orifices and/or by decreasing the polymer flow rate or by increasing gas withdrawal behind the collecter.

The oil absorbing wipes formed from the consolidated film-like fiber nonwoven webs are such that the wipe generally has a void volume of from 40 to 80 percent, preferably 45 to 75 percent and most preferably 50 to 70 percent. Where the void volume is greater than 70 percent it is difficult to obtain a rapid change in transparency or opacity as large amounts of oil are necessary to create this change, also the material becomes to compliant and difficult to handle. Where the void volume is less than 40%, the material becomes too stiff and has an insufficient capacity to absorb oil. The average pore size of the wipe is generally from 3-15 microns, preferably 3 to 12 microns and most preferably 4 to 8 microns. If the pore size is less than 3 microns, it is difficult to get the rapid oil absorption rate needed. Void volume and pore size generally can be decreased by higher consolidation conditions and/or decreasing the average fiber diameter or narrowing the range of fiber diameters. If the pore size is greater than 15 microns the ability to retain absorbed oil is lessened as is the rapid oil indicating function. Generally the void volume, basis weight and pore size should be provided to yield an oil absorption capacity of from 0.7 to 6 mg/cm$^2$, preferably 0.8 to 5 mg/cm$^2$ and most preferably 0.9 to 4 mg/cm$^2$. If the oil absorption is less than this then the capacity to absorb facial oil is insufficient for most users and when greater than these levels then the rapid oil absorption indicating function is adversely impacted for most users.

A preferred thermoplastic material for forming the web fibers is polypropylene wherein the desired initial and end opacity for a given wipe is controlled by the basis weight of the web forming the wipe material, the hardness of the calendering rolls, and the calendering (or consolidation) pressure and temperature. Generally, for polypropylene, a web or wipe basis weight of about 10 g/m$^2$ to 40 g/m$^2$ has been found suitable to provide an adequate initial transparency while allowing a change in transparency at a suitably low oil loading level with a relatively soft hand. Generally, the Hand of the wipe should be 8 grams or less, preferably 1-7 grams and most preferably 1-6 grams. For polypropylene wipes, basis weights of greater than about 40 g/m$^2$ are too stiff to be useful as a facial wipe. For fibers formed of other polymers or polymer blends under similar calendering conditions, different wipe basis weight ranges may be suitable depending on the oil absorbing properties and relative stiffness of the fibers forming the web.

Higher calendering temperatures and pressures have been found to have significant effects on the original transparency, pore size and void volume and also the resulting oil absorption capacity of the consolidated wipe. Higher calendering temperatures in particular significantly increase the original transparency, thus decreasing the oil-indicating value of the wipe. Under certain circumstances, it would be desirable to use chilled calendering rolls to counteract this effect However, when a web is over-calendered (e.g., under too high a pressure and/or temperature), the web does not become more rigid, however, the oil indicating function and absorption capacity does decrease.

If the original opacity is inadequate to produce a significant enough change in opacity, opacifying agents such as silica, talc, calcium carbonate or other like inorganic powders can be used at low levels. Such powders could be coated on the surface of the wipes or incorporated into the web structures. Suitable methods for incorporating opacifying agents into the web include that taught in U.S. Patent No. 3,971,373 where a stream of particles is entrained into two separate converging melt-blown microfiber streams prior to collection. Another method of incorporating particulates is taught in U.S. Patent No. 4,755,178 where particles are introduced into an airstream that converges into a flow of melt-blown microfibers. Preferably, only a small amount of such opacifying agents are included as they have the tendency to detract from the wipe softness.

In addition to the above, other conventional web additives such as surfactants, colorants, and antistatic agents can be incorporated into the web by known methods.

The invention oil absorbent wipes are generally characterized by the ability to change from opaque to translucent after absorbing only a moderate amount of oil, such as would be present on a person's skin (e.g., from 0 to 8 mg/cm$^2$). The oil absorbent wipes are particularly useful as cosmetic wipes as after absorbing skin oil at the levels excreted from common sebaceous glands, they will turn translucent, thus indicating that the undesirable oil has been removed and that makeup or other skin treatments can be applied. The oil-indicating effect is provided by an oil absorbing wipe having an initial transparency of about 65 percent or less, preferably 60 percent or less with an ability to change transparency by about 30 percentage points or more, preferably 35 percentage points or more with a relatively low level of oil loading (e.g., 6 mg/cm$^2$). The oil absorbing wipe is generally used as a single layer material but could be laminated to other like web materials, or films or the like.

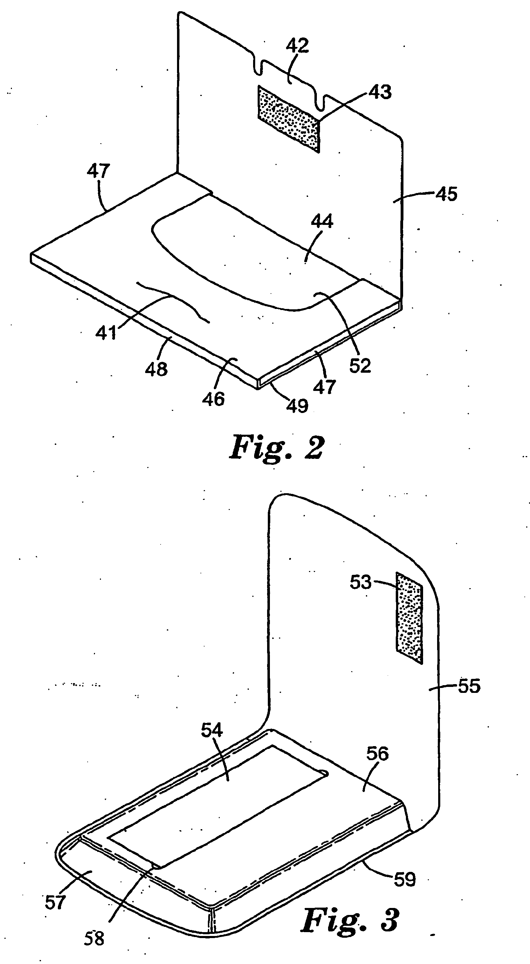

Referring to Fig. 2, a dispensable package of oil wipes in accordance with the invention comprises a dispensable package including individual wipes 44 of oil absorbent wipe material. The package generally comprises a top wall 46 and bottom wall 49, generally parallel to one another, and two side walls 47. A front edge 48 is provided where the back edge is formed into a flap 45, which can be folded down onto the top wall 46 of the package. The flap 45 can engage with the package by use of an adhesive 43 or the like, provided as is known in the art. Alternatively, a tab 42 engagable within a slot 41 can be used as a macro-mechanical type closure. Other conventional methods known in the art include the use of cohesive materials, hook and loop fasteners, living hinges, snaps and the like to keep the flap 45 in place to cover the access opening 52 to the wipes. The dispensable package contains an access opening 52 which permits a user to grasp an individual wipe and withdraw it from the package for use. Generally, the access opening 52 is at its largest dimension smaller than the largest length or width dimension of the dispensable oil absorbing wipe material or wipe. However, if the individual wipes are connected in a manner that they are separable from one another then the access opening should be as large or smaller than the dimension of the wipe which is pulled through the access opening.

The discrete wipe materials can be either separated from one another or separable from one or another, both are considered to be discrete wipes or wipes according to the invention. Generally, separable wipes are provided by having a frangible connection between the discrete wipes which allow the user to break and to separate the discrete wipes one from the other. Frangible connections can be created by lines of weaknesses such as perforations, score lines or by the use of additional weak adhesive-type attachment materials or by simply frictional engagement. Discrete separate wipes would require no breaking of a frangible connection. The wipes further can be stacked, provided in a roll, or folded and the like as is conventionally known for tissue-type papers. Folding is generally provided by an interleaving arrangement via v-folds, z-folds or the like. With this type of folding, opposing overlapping ends of adjacent wipes allow removal of an upper wipe to provide the lower wipe in an engagable form by frictionally pulling the lower wipe up and out through an access opening for subsequent use.

An alternative embodiment of a dispensable package arrangement is shown in Fig. 3, the top wall portion 56 is provided with an access opening slot 58 through which a wipe 54 of oil absorbent wipe material is graspable. In this embodiment, the discrete wipes of wipe material must be interconnected so that the upper wipe can pull the lower wipe up and through the opening slot 58. This interconnection can be by separate wipes that are folded in an interleaving manner as described above. Alternatively the wipes could be separable wipes as described above; for example; separable wipes can be interconnected through a frangible connection. The movable flap 55 is provided on a sidewall portion and, like the flap in the Fig. 2 embodiment, can be provided with a suitable closure element 53, such as a patch of pressure-sensitive adhesive.

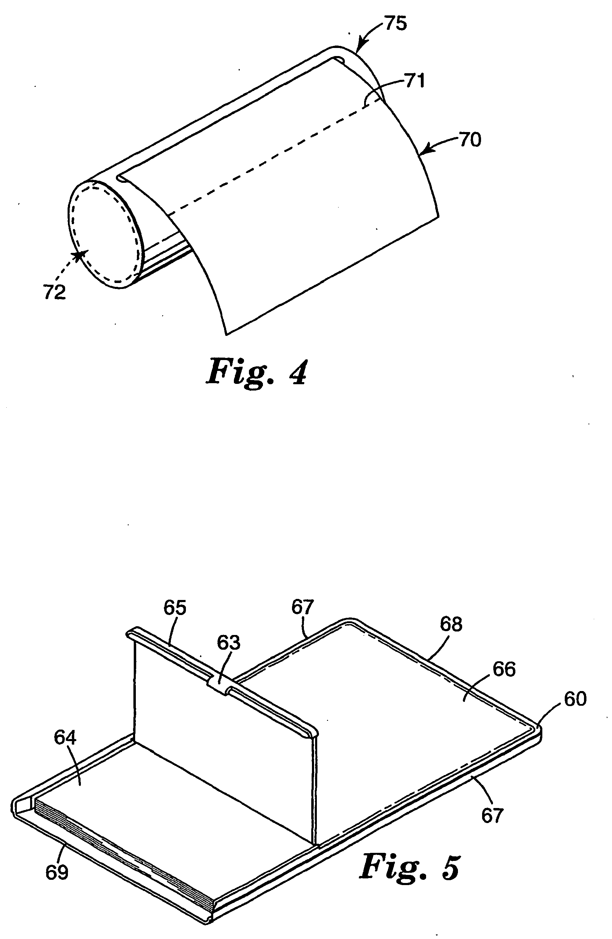

A further alternative embodiment of the dispensable oil absorbent wipe package is shown in Fig. 4 which shows a roll of discrete wipe materials 70 connected by frangible connections 71 which can be rolled into a roll form 72, with or without a core, allowing the materials to be grasped and dispensed from a roll dispenser 75.

Fig. 5 shows an alternative embodiment of a dispensable package of the oil absorbent wipes formed with a rigid frame container 60, preferably thermoplastic. The individual wipe materials 64 are contained within the container 60, which has a top wall 66 containing a movable flap 65, which is generally movable by a living hinge. A clasp 63 is provided on the outermost end of flap 65, which clasp 63 engages with the bottom wall 69 to provide for closure of the container 60. Side walls 67 contain the wipes 64 within the container 60 coupled with the top wall 66 and bottom wall 69. End wall 68 is preferably closed. In this embodiment, the individual wipes of discrete oil absorbent material would generally be stacked as separate wipes in an overlying stack preferably of coextensive wipes. The user would grasp an individual wipe and remove, each one separately from the container using the frictional force of their fingers to separate the upper wipe from the immediate lower wipe. The individual wipes would then be used to remove skin oil by wiping over the user's face. Following use, the wipe is easily compacted into a small volume shape for easy disposal.

The individual discrete wipes can be of any suitable size, however, generally for most applications the wipes would have an overall surface area of from 10 to 100 cm$^2$, preferably from 20 to 50 cm$^2$. As such, the wipes would be of a size suitable for insertion in a package, which could easily be placed in the user's purse or pocket. The material forming the dispensable containers is generally not of importance and can be formed of suitable papers, thermoplastics, paper film laminates and the like. The shape of the tissues is generally rectangular, however, other suitable shapes such as oval, circular or the like can be used.

The oil-absorbing wipes of the invention can be coated with any active or nonactive ingredients or agents in the film forming polymeric coating. Additional ingredients can comprises a wide range of optional ingredients. Particularly useful are various active ingredients useful for delivering various benefits to the skin or hair during and after oil removal and cleansing.

The film forming coating compositions of the present invention can include a safe and effective amount of one or more pharmaceutically-acceptable active or skin modifying ingredients. The term "safe and effective amount" as used herein, means an amount of an active ingredient high enough to modify the conditions to be treated or to deliver the desired skin benefit, but low enough to avoid serious side effects. What is a safe and effective amount of the active ingredient will vary with the specific active ingredient, the ability of the active ingredient to penetrate through the skin, the age, health condition, and skin condition of the user, and other like factors.

The active ingredients useful herein can be categorized by their therapeutic benefit or their postulated mode of action. However, it is to be understood that the active ingredients useful herein can in some instances provide more than one therapeutic benefit or operate via more than one mode of action. The following active ingredients are useful in the compositions used in the present invention. Anti-acne actives: examples of useful anti-acne actives include the keratolytics such as salicylic acid (o-hydroxybenzoic acid), derivatives of salicylic acid, retinoids such as retinoic acid and its derivatives (e.g., cis and trans); sulfur-containing D and L amino acids and their derivatives and salts, lipoic acid; antibiotics and antimicrobials; sebostats such as flavonoids; and bile salts such as scymnol sulfate and its derivatives, deoxycholate, and cholate Anti-wrinkle and anti-skin atrophy actives: examples of antiwrinkle and anti-skin atrophy actives include retinoic acid and its derivatives (e.g., cis and trans); retinol; retinyl esters; niacinamide, salicylic acid and derivatives thereof; sulfur-containing D and L amino acids and their derivatives and salts, thiols, hydroxy acids phytic acid, lipoic acid;lysophosphatidic acid, and skin peel agents (e.g., phenol and the like). Non-steroidal anti-inflammatory actives (NSAIDS): examples of NSAIDS include the following, propionic acid derivatives; acetic acid derivatives; fenamic acid derivatives; biphenylcarboxylic acid derivatives; and oxicams. Topical anesthetics; examples of topical anesthetic drugs include benzocaine, lidocaine, bupivacaine, chlorprocaine, dibucaine, etidocaine, mepivacaine, tetracaine, dyclonine, hexylcaine, procaine, cocaine, ketamine, pramoxine, phenol, and pharmaceutically acceptable salts thereof. Artificial tanning agents and accelerators; examples of artificial tanning agents and accelerators include dihydroxyacetaone, tyrosine, tyrosine esters such as ethyl tyrosinate, and phospho-DOPA. Sunscreen actives; examples of sunscreens which are useful in the compositions of the present invention are those selected from the group consisting of 2-ethylhexyl p-methoxycinnamate, 2-ethylhexyl NN-dimethyl-p-aminobenzoate, p-aminobenzoic acid, 2-phenylbenzimidazole-5-sulfonic acid, octocrylene, oxybenzone, homomenthyl salicylate, octyl salicylate, 4,4'-methoxy-t-butyldibenzoylmethane, 4-isopropyl dibenzoylmethane, 3-benzylidene camphor, 3-(4-methylbenzylidene) camphor, titanium dioxide, zinc oxide, silica, iron oxide and mixtures thereof. Other known active agents such as antibiotics or antiseptics may also be used.

The coating compositions used in the present invention can comprise a wide range of other components which can provide skin benefits, or modify the skin or modify the coating composition. These additional components should generally be pharmaceutically acceptable. The *CTFA Cosmetic Ingredient Handbook,* Second Edition, 1992, describes a wide variety of nonlimiting cosmetic and pharmaceutical ingredients commonly used in the skin care industry, which may be suitable for use in the coating compositions of the present invention. Nonlimiting examples of classes of ingredients are described at page 537 of this reference. Examples of these and other classes include: fillers, abrasives, absorbents, anticaking agents, antioxidants, vitamins, binders, biological additives, buffering agents, bulling agents, chelating agents, chemical additives, colorants, cosmetic astringents, cosmetic biocides, denaturants, drug astringents, external analgesics, film formers, fragrance components, humectants, opacifying agents, pH adjusters, preservatives, propellants, reducing agents, skin bleaching agents, and sunscreening agents. Preferred are particulate additives that can be used to act as opacifiers, pigments or fillers. Suitable inorganic pigments or fillers would be talc, titanium dioxide, calcium carbonate and zinc oxide.

Also useful as additives are aesthetic components such as fragrances, pigments, colorings, essential oils, skin sensates, astringents, skin soothing agents, and skin healing agents.

Suitable preferred film forming agents for forming the film forming coating include film-forming polymers such as polyvinyl alcohol, dimethicone copolyol, poly(N-vinyl formamide), polyacrylamide, poly(hydroxyethyl), methacrylate polyethylene oxide, polyvinyl pyrrolidone, polyvinyl acetate, polyhydroxylalkyl cellulose ethers such as carboxymethylcellulose, 2-hydroxyethyl cellulose, 2-hydroxyethylmethyl cellulose or 2-hydroxypropyl cellulose.

Water insoluble film forming polymers suitable for this invention include, but are not limited to, water insoluble polyamide polymers; esters of polymeric carboxylic acids, e.g., polyacrylate polymers; polypropylene oxide and derivatives thereof; and the like.

Water soluble film-forming polymers may be cationic, anionic or nonionic polymers. Preferred water soluble film forming polymers include: cellulose derivatives such as quaternary nitrogen-containing cellulose ethers, hydroxyethyl cellulose, hydroxypropyl cellulose and hydroxyethyl alkali metal carboxylalkyl cellulose derivatives, and free acid hydroxyalkyl carboxyalkyl cellulose derivatives; polyvinyl alcohol; vinylpyrrolidone homopolymers and copolymers; polycarboxylic acid derivatives; polyacrylamides; vinyl methyl ether homopolymers and copolymers; ethylene oxide resins; and the like.

The choice of film-forming polymer is not critical and may comprise any of the above types of water soluble or water insoluble film-forming polymers or blends thereof. However, the film-forming polymer when applied to the oil absorbent wipe must be in solution with an evaporative solvent to provide for effective penetration of the coating into the oil-absorbing wipes such that the film forming polymer is at least partially penetrated into the wipe when the evaporative solvent is removed. The film-forming polymer may be completely or partially soluble in the solvent and the solvent must at least in part wet the oil absorbent wipe. However, if the film-forming polymer is only partially soluble, the concentration of the film-forming polymer should be below the saturation level so that all the film-forming polymer is at least partially soluble in the solvent, the saturation level must also be sufficient, i.e., high enough, to provide at least the minimum weight ratio of polymer to other active agents or ingredients. The viscosity of the film forming polymer coating or solution is generally at least 10 cps (0.01 Pa·s) and from 2000 to 10,000 cps (2 to 10 Pa·s) for coating porous film substrate, preferably 3000 to 50,000 cps (3 to 5 Pa·s). With consolidated nonwoven type substrates the viscosity of the of the film forming polymer coating is generally from 10,000 to 100,000 cps (10 to 100 Pa·s) cps and preferably 15,000 to 50,000 cps (15 to 5 Pa·s). The use of too low a viscosity solution would cause excessive penetration into the porous wipe material making the coating visible from the opposite face. Too high a viscosity can result in too low a bond between the coating and the oil absorbent wipe material resulting in the coating falling off or cracking and increasing the difficultly in coating. The percent solids in the coating solution is generally 50 to 80, preferably 60 to 70 for porous film substrates with consolidated nonwoven substrate the percent solids is generally 50 to 80, preferably 65 to 75. The viscosity and percent solids is adjusted to the desired levels by use of the solvent, fillers or viscosity modifying agents. The choice of solvent and percent solids can be used to adjust the viscosity and penetration of the solution into the porous wipe.

The amount of particulates in the solids is adjusted depending on the film forming polymer. The dried coating following evaporation of the solvents and other volatile components is generally a film forming polymer with about 35 to 55 percent by weight particulate filler to film forming polymers and other nonparticulate solids in the coating, preferably 40 to 50 percent particulate filler to other solids in the coating. Additional active agents or other ingredients make up the remainder of the dried coating. This generally provides a nontacky but flexible or non brittle film depending on the nature of the film forming polymer. The exact amount of filler useable with a particular film forming polymer depends on the nature of the film former and the size of the particulate filler. Generally, the particulate filler is a solid having an average size of from 0.1 to 30 microns. If too low an amount of filler is used, the coating becomes tacky and causes adjacent wipes to block or adhere to each other in a package form. If too high an amount of filler is used, the coating becomes brittle and falls off to easily. If a coating falls off this would result in either no coating over a portion of the wipe that has been coated or a nonuniform distribution of the coating over the area of the oil absorbent wipe that has been coated. For functionality purposes, it is preferred that there is a substantially uniform distribution of a continuous or pattern coating over a substantial portion of one face of the wipe, generally 10 to 100 percent of one face of the wipe, preferably 50 to 100 percent. If the coating is a discontinuous pattern it can be applied in any suitable pattern such as dots, lines, discrete patterns, logos, etc., as would be known in the art.

The penetration of the film forming polymer coating into the oil absorbent wipe is from 10 to 90 percent of the wipe thickness and most preferably from 20 to 80 percent of the wipe thickness. If the percent penetration is too high, then the user will not be able to differentiate the oil absorbent side from the polymer coated side of the wipe. Further, too high a degree of penetration of the film forming polymer into the wipe will significantly impair the oil absorbency functionality of the oil absorbent wipe. If the film forming polymer has little or no penetration into the wipe then the coating can easily be removed or fall off the wipe surface.

The evaporative solvent used with the film forming polymer may be any liquid in which the film-forming polymer is completely or substantially soluble and which will readily evaporate when used in compositions suitable for the present invention. The evaporation characteristics of the evaporative solvent can usually be characterized based on the partial vapor pressure of such solvents. The partial pressure of evaporative solvents of the present invention will generally be greater than 1, preferably from about 10 mm to about 250 mm at 25°C. The partial pressure of many compounds may be determined experimentally using standard procedures as reported in literature. Preferred solvents include alcohols and water.

Organic gelling agents can be employed as viscosity modifiers in amounts of from about 5% to 25% and comprise those of natural or synthetic origin. Preferred gelling agents are starches such as the glycerol starches, microcrystalline cellulose and hydroxyalkyl cellulose ethers such as hydroxypropylmethyl cellulose (HPMC), hydroxymethyl cellulose (HMC), carboxymethyl cellulose (CMC), 2-hydroxyethyl cellulose, 2-hydroxyethylmethyl cellulose, propylene carbonate and 2-hydroxypropyl cellulose (Klucel® H); or one or more short carbon chain alcohols such as ethanol, isopropyl alcohol, propylene glycol, butylene glycol, hexylene glycol, polyethylene glycol, methoxypolyethylene glycol and their derivatives.

One embodiment of a preferable method for manufacturing the wipe pack of the present invention is as follows. The film forming polymer with the active or inactive skin modifying substances or other additives and fillers are homogeneously stirred, and a solvent is added thereto to adjust the viscosity, to give a coatable polymer solution or flurry. In this case, it is preferable that the solvent content is 25% to 50% by weight. Thereafter, the film forming polymer coating solution or slurry is uniformly spread directly onto an oil absorbing wipe substrate with an applicator continuously or in a pattern, preferably without any intermediate layer such as a fibrous layer. The solvent is subsequently actively evaporated by use of heat, reduced pressure or the like or allowed to evaporate in the air. The coated oil absorbing wipe substrate is then cut into discrete pieces or used as a continuous piece with frangible connections and provided into a package as is described above.

Test Methods

Viscosity

The viscosities of the coatings used for the facial wipes of the invention were measured using a Brookfield Viscometer. Spindle 2 was used at 1.5 rpm for all measurements except where noted. Results are reported in centipoise (cps); 1 cps corresponds to 0.001 Pa·s.

Visual Appearance

A determination was made for each of the examples and counter-examples as to whether the coatings had soaked through the wipe and thus became visible from the uncoated side of the wipe as noted with a 'Y' (Yes) or an 'N' (No) in the following tables. Visibility of the coating from the uncoated side of the wipe is not desirable, as this may interfere with the users ability to detect sebum absorption from the wiping of their skin.

Examples

Examples 1-9

Multi-functional facial wipes were prepared using the coatings shown below in Table 1 (all parts by weight) and a microporous film similar to that described in PCT application WO 99/29220 Example 1 as a substrate, having the following composition: 5D45 polypropylene (62.9%, Union Carbide Co.), mineral oil (35.0%, white oil #31, Amoco Oil & Chemical Co.), iron oxide red pigment (2.0%, CI #77491 russet, Sun Chemical Co.) and Millad® 3988 nucleating agent (0.1%, Milliken Chemical). The microporous film had a thickness of 37 microns and a void content of 30%. The coatings were prepared by weighing the listed ingredients followed by mixing and homogenizing by hand or mechanical shaker. The coatings were then diluted with isopropyl alcohol to various percent solids. For simplicity, all ingredients other than the isopropyl alcohol were considered solids. This same definition of percent solids is used for all purposes in the invention. The microporous films were coated using the following technique. A 7 cm x 10 cm piece of microporous film was laid onto a flat surface. A thermoplastic screen constrained in an embroidery hoop, having 15 - 1mm diameter perforations/cm$^2$, was placed over the microporous film. A modest quantity of the coating solution was placed onto the screen. The coating was forced through the screen opening with a thermoplastic scraper onto the microporous film, resulting in a dot coating. The coated microporous film was then allowed to air dry for 24 hours before making observations.

Table 1

| Component | Example | | | | | | | | |
|---|---|---|---|---|---|---|---|---|---|
| | 1 | 2 | 3 | 4 | 5 | 6 | 7 | 8 | 9 |
| PVP-1[(1)] | 46 | 46 | | | | | | | |

(continued)

| Component | Example | | | | | | | | |
|---|---|---|---|---|---|---|---|---|---|
| | 1 | 2 | 3 | 4 | 5 | 6 | 7 | 8 | 9 |
| PVP-2[2] | | | 46 | 46 | 46 | 46 | | | |
| PVP-3[3] | | | | | | | 46 | 46 | 46 |
| Butylene glycol[4] | 6 | 6 | 6 | 6 | 6 | 6 | 6 | 6 | 6 |
| Talc[5] | 46 | 46 | 46 | 46 | 46 | 46 | 46 | 46 | 46 |
| Salicylic acid[6] | 2 | 2 | 2 | 2 | 2 | 2 | 2 | 2 | 2 |
| % Solids | 70 | 75 | 60 | 65 | 70 | 75 | 60 | 65 | 70 |
| Viscosity | 3500 | 22500 | 3500 | 13250 | 37750 | 274000[7] | 3250 | 15750 | 37750 |
| % Particulates after drying | 46 | 46 | 46 | 46 | 46 | 46 | 46 | 46 | 46 |
| Did coating soak thru to noncoated side? (Y/N) | N | N | N | N | N | N | N | N | N |

(1) polyvinylpyrrolidone -10,000 Mw, Aldrich Chemical Co.
(2) polyvinylpyrrolidone - 40,000 Mw, Povidone K-30, BASF Chemical Co.
(3) polyvinylpyrrolidone - 55,000 Mw, Aldrich Chemical Co.
(4) butylene glycol - 1,3-butanediol, Aldrich Chemical Co.
(5) talc - Aldrich Chemical Co.
(6) salicylic acid - Aldrich Chemical Co.
(7) measured with spindle 4 at 1.5 rpm

Examples 10-19

Multi-functional facial wipes were prepared as in Examples 1-9 above except several different glycols were used at various percent solids to demonstrate possible variations in coating compositions as shown in Table 2 below.

Table 2

| Component | Example | | | | | | | | | |
|---|---|---|---|---|---|---|---|---|---|---|
| | 10 | 11 | 12 | 13 | 14 | 15 | 16 | 17 | 18 | 19 |
| PVP-1 | 46 | 46 | 46 | 46 | 46 | 46 | 46 | 46 | 46 | 46 |
| PBG-1[1] | 6 | 6 | | | | | | | | |
| PEG-2[2] | | | 6 | 6 | | | | | | |
| PEG-3[3] | | | | | 6 | 6 | | | | |
| HG-4[4] | | | | | | | 6 | 6 | 6 | |
| MMBG-1[5] | | | | | | | | | | 6 |
| Talc | 46 | 46 | 46 | 46 | 46 | 46 | 46 | 46 | 46 | 46 |
| Salicylic acid | 2 | 2 | 2 | 2 | 2 | 2 | 2 | 2 | 2 | 2 |
| % Solids | 70 | 75 | 65 | 75 | 70 | 75 | 65 | 70 | 75 | 75 |
| Viscosity | 3000 | 27750 | | 32250 | 9550 | 34750 | 2250 | 4000 | 23250 | 32750 |
| % Particulates after drying | 46 | 46 | 46 | 46 | 46 | 46 | 46 | 46 | 46 | 46 |

(continued)

|  | Example | | | | | | | | | |
|---|---|---|---|---|---|---|---|---|---|---|
| Component | 10 | 11 | 12 | 13 | 14 | 15 | 16 | 17 | 18 | 19 |
| did coating soak thru to noncoated side? (Y/N) | N | N | N | N | N | N | N | N | N | N |
| (1) polyethylene glycol - 600 Mw, Aldrich Chemical Co.<br>(2) polyethylene glycol -1500 Mw, Aldrich Chemical Co.<br>(3) polyethylene glycol - 2000 Mw, Aldrich Chemical Co.<br>(4) hexylene glycol-1,6-hexanediol, Aldrich Chemical Co.<br>(5) 3-methoxy 3-methyl 1-butanol - Kuraray Chemical Co. | | | | | | | | | | |

Examples 20-25

Muiti-functional facial wipes were prepared as in Examples 10-19 above except a 40,000 Mw PVP-2 was used as the film-forming polymer. Several different glycols were used at various percent solids to demonstrate possible variations in coating compositions as shown in Table 3 below.

Table 3

|  | Example | | | | | |
|---|---|---|---|---|---|---|
| Component | 20 | 21 | 22 | 23 | 24 | 25 |
| PVP-2 | 46 | 46 | 46 | 46 | 46 | 46 |
| PPG-1[(1)] | 6 | | | | | |
| PEG-1 | | 6 | | | | |
| PEG-2 | | | 6 | | | |
| PPG-2[(2)] | | | | 6 | | |
| HG-1 | | | | | 6 | |
| MMBG-1 | | | | | | 6 |
| Talc | 46 | 46 | 46 | 46 | 46 | 46 |
| Salicylic acid | 2 | 2 | 2 | 2 | 2 | 2 |
| % Solids | 60 | 60 | 60 | 65 | 60 | 60 |
| Viscosity | 3750 | 3500 | --- | 15000 | 3750 | 3250 |
| % Particulates after drying | 46 | 46 | 46 | 46 | 46 | 46 |
| Did coating soak thru to noncoated side? (Y/N) | N | N | N | N | N | N |
| (1) polypropylene glycol - 400 Mw; Matheson,Coleman & Bell Chemical Co.<br>(2) polypropylene glycol - 2000 Mw, Aldrich Chemical Co. | | | | | | |

Examples 26-35

Multi-functional facial wipes were prepared as in Examples 20-25 above except several different ingredients were used in place of salicylic acid to demonstrate possible variations in coating compositions as shown in Table 4 below.

Table 4

|  | Example | | | | | | | | | |
|---|---|---|---|---|---|---|---|---|---|---|
| Component | 26 | 27 | 28 | 29 | 30 | 31 | 32 | 33 | 34 | 35 |
| PVP-2 | 46 | | 46 | | 46 | | 46 | | 46 | |
| PVP-3 | | 46 | | 46 | | 46 | | 46 | | 46 |

(continued)

| Component | Example | | | | | | | | | |
|---|---|---|---|---|---|---|---|---|---|---|
| | 26 | 27 | 28 | 29 | 30 | 31 | 32 | 33 | 34 | 35 |
| Butylene glycol | 6 | 6 | 6 | 6 | 6 | 6 | 6 | 6 | | |
| Talc | 46 | 46 | 46 | 46 | 46 | 46 | 46 | 46 | 46 | 46 |
| Aloe gel[1] | 2 | 2 | | | | | | | | |
| Lanolin[2] | | | 2 | 2 | | | | | | |
| Almond oil[3] | | | | | 2 | 2 | | | | |
| Ascorbic acid[4] | | | | | | | 2 | 2 | | |
| Glitter[5] | | | | | | | | | 2 | 2 |
| % Solids | 65 | 65 | 65 | 65 | 65 | 65 | 65 | 65 | 65 | 65 |
| Viscosity | 9500 | 13250 | 13750 | 16250 | 12750 | 15300 | | 23750 | 12250 | 17250 |
| % Particulates after drying | 46 | 46 | 46 | 46 | 46 | 46 | 46 | 46 | 48 | 48 |
| Did coating soak thru to noncoated side? (Y/N) | N | N | N | N | N | N | N | N | N | N |
| (1) AloeVera 80 - Naturade<br>(2) Lanolin - Fisher Scientific Co.<br>(3) Almond oil - Hain Pure Food Co.<br>(4) Ascorbic acid - Mallinckrodt Chemical Co.<br>(5) Ultrabrite™ glitter .0115 Hex - Glitterex Corp. | | | | | | | | | | |

Examples 36 - 37

Multi-functional facial wipes were prepared as in Examples 26-35 above except aspirin (acetylsalicylic acid) and zinc oxide were used as ingredients in combination with a 10,000 Mw PVP to demonstrate possible variations in coating compositions as shown in Table 5 below.

Table 5

| Component | Example | |
|---|---|---|
| | 36 | 37 |
| PVP-1 | 46 | 46 |
| Butylene glycol | 6 | 6 |
| Talc | 46 | 46 |
| Aspirin[1] | 2 | |
| Zinc oxide[2] | | 2 |
| % Solids | 75 | 75 |
| Viscosity | | |
| % Particulates after drying | 46 | 48 |
| Did coating soak thru to noncoated side? (Y/N) | N | N |
| (1) acetylsalicylic acid - commercial aspirin<br>(2) zinc oxide - Aldrich Chemical Co. | | |

Examples 38-46

Multi-functional facial wipes were prepared as in Examples 1-9 above except different film-forming polymers were used in place of polyvinylpyrrolidone to demonstrate possible variations in coating compositions as shown in Table 6 below.

Table 6

| Component | Example | | | | | | | | |
|---|---|---|---|---|---|---|---|---|---|
| | 38 | 39 | 40 | 41 | 42 | 43 | 44 | 45 | 46 |
| QVP(1) | 46 | 46.7 | | 32.2 | | | | | |
| PVP-1 | | | | | 10 | 15 | 20 | 26 | 26 |
| PEI(2) | | | | | 36 | 31 | 26 | 18 | 15 |
| PVP/VA(3) | | | 46 | 24.2 | | | | | |
| Butylene glycol | 6 | 5 | 6 | | 6 | 6 | 6 | 6 | 6 |
| Pharma-Solve(4) | | | | 1.7 | | | | | |
| Talc | 46 | 46.7 | 46 | 40.2 | 46 | 46 | 46 | 46 | 46 |
| Salicylic acid | 2 | 1.7 | 2 | | 2 | 2 | 2 | 2 | 2 |
| Span 20(5) | | | | | | | | 2 | |
| Glycerol(6) | | | | | | | | | 5 |
| % Solids | 70 | 50 | 60 | 50 | 78 | 78 | 78 | 78 | 78 |
| Viscosity | 3250 | 3630 | 1720 | | | | | | |
| % Particulates after drying | 46 | 46.7 | 46 | 40.2 | 46 | 46 | 46 | 46 | 46 |
| Did coating soak thru to noncoated side? (Y/N) | N | N | N | N | N | N | N | N | N |

(1) GAFQUAT® 734 - Quarternized vinylpyrrolidone/Dimethylaminoethylmethacrylate copolymer-100,000 Mw, 50% ethanol solution, ISP Technologies Inc.
(2) polyethylenimine - 750,000 Mw, 50% aqueous solution, Aldrich Chemical Co.
(3) Plasdone® S-630 - vinylacetate/N-vinylpyrrolidone copolymer, ISP Technologies Inc.
(4) N-methyl-2-pyrrolidone, ISP Technologies
(5) Span 20- sorbitan monolaurate, Ruger Chemical Co. Inc.
(6) Glycerol, Mallinckrodt Inc.

Comparative Examples C1-C4

The compositions of Examples 3-6 were coated onto two commercially available facial oil removing paper tissues available from the Kose Co. and Yojiya Co. of Japan using the same coating technique as in the above examples. The results in Table 7 below show that at low percent solids and viscosities, the coatings soaked through the tissues and were visible on the uncoated side of the tissue papers.

Table 7

| | Counter Examples | | | |
|---|---|---|---|---|
| | Paper Tissue 1(1) | | Paper Tissue 2(2) | |
| Component | C1 | C2 | C3 | C4 |
| PVP-2 | 46 | 46 | 46 | 46 |
| Butylene glycol | 6 | 6 | 6 | 6 |
| Talc | 46 | 46 | 46 | 46 |
| Salicylic acid | 2 | 2 | 2 | 2 |

(continued)

|  | Counter Examples | | | |
|---|---|---|---|---|
|  | Paper Tissue 1[1] | | Paper Tissue 2[2] | |
| % Solids | 60 | 65 | 60 | 65 |
| Viscosity | 3500 | 13250 | 3500 | 13250 |
| % Particulates after drying | 46 | 46 | 46 | 46 |
| Did coating soak thru to noncoated side? (Y/N) | Y | Y | Y | Y |
| (1) facial blotting tissue - Kose Co. of Japan<br>(2) facial blotting tissue - Yojiya Co. of Japan | | | | |

Examples 47 - 50

The compositions used in Table 7 above were coated onto the same paper tissues as in C1 - C4 except the viscosity of the compositions was increased by increasing the percent solids. The results in Table 8 below show that at higher percent solids and viscosities, the coatings do not soak entirely through the paper tissues, although the coatings were still visible on the uncoated side of the tissue papers.

Table 8

|  | Examples | | | |
|---|---|---|---|---|
|  | Paper Tissue 1 | | Paper Tissue 2 | |
| Component | 47 | 48 | 49 | 50 |
| PVP-2 | 46 | 46 | 46 | 46 |
| Butylene glycol | 6 | 6 | 6 | 6 |
| Talc | 46 | 46 | 46 | 46 |
| Salicylic acid | 2 | 2 | 2 | 2 |
| % Solids | 70 | 75 | 70 | 75 |
| Viscosity | 37750 | 274000[1] | 37750 | 274000[1] |
| % Particulates after drying | 46 | 46 | 46 | 46 |
| Did coating soak thru to noncoated side? (Y/N) | N | N | N | N |
| (1) spindle 4 at 1.5 RPM | | | | |

Comparative Examples C5-C9

It is well known in the patent art to impregnate tissues and nonwovens with medicated, cleansing, or cosmetic compositions. Several compositions from the wipe patent art were selected and coated onto the microporous films of Examples 1-46 using the same coating technique as in the above examples. The results in Table 9 below show that known compositions from the patent art do not work when coated onto the microporous films used in the invention because they soak through the film and are visible from the uncoated side, or the coatings are invisible on the coated side, or are extremely stiff after drying. C9 was prepared by taking the composition of Examples 1 and 2 and diluting it to 60% solids to demonstrate that low viscosity coatings soak through the film.

Table 9

|  | Comparative example | | | | |
|---|---|---|---|---|---|
| Component | C5[a] | C6[b] | C7[c] | C8[d] | C9[e] |
| PVA[1] |  | 9.5 | 9.5 |  | 46 |
| Glycerol[2] |  | 19.0 | 19.0 |  |  |

(continued)

| | Comparative example | | | | |
|---|---|---|---|---|---|
| Butylene glycol | | | | | 6 |
| Petrolatum(3) | 34.6 | | | | |
| Salicylic acid | | | | 1.5 | |
| Citric acid(4) | 20.1 | | | | |
| Propanediol(5) | 10.0 | | | | |
| Talc | | | 42.9 | | 46 |
| Kaolin clay(6) | | 42.9 | | | |
| Hexadecanol(7) | 10.1 | | | | |
| Octadecanol(8) | 25.2 | | | | |
| Ethanol(9) | | | | | |
| Distilled water | | 28.6 | 28.6 | 72.7 | |
| Aloe Vera(10) | | | | 0.4 | |
| % Solids | 54.7 | 71.4 | 71.4 | 1.9 | 60 |
| Viscosity | | | | | 375 |
| % Particulates after drying | | 60 | 60 | 100 | 46 |
| Did coating soak thru to noncoated side? (Y/N) | Y | N coating was brittle | N coating was brittle | N coating was not visible | Y |
| (a) US Patent 5,830,487 Example 1<br>(b) US Patent 4,569,343 Example 1 lines 55-60<br>(c) Same as (b) except talc was substituted for clay<br>(d) U.S. Patent 5,744,149 Example VI<br>(e) Example 1 except 60% solids<br>(1) Polyvinyl alcohol 10,00OMw - Aldrich Chemical Co.<br>(2) Glycerol- Mallinckrodt Inc<br>(3) Petrolatum_E.M. Science<br>(4) Citric acid_-E.M. Science<br>(5) Propanediol- Aldrich Chemical Co.<br>(6) Kaolin clay- Aldrich Chemical Co.<br>(7) Hexadecanol- Aldrich Chemical Co.<br>(8) Octadecanol- Aldrich Chemical Co.<br>(9) Ethanol- E.M. Science<br>(10) Aloe Vera- Fruit of the Earth Inc. | | | | | |

Examples 51-54

To demonstrate the effect of the coatings used in the invention on other microporous films, the same composition and coating technique used in Example 2 was applied to four commercially available microporous films(MPF) as shown in Table 10.

Table 10

| | Example | | | |
|---|---|---|---|---|
| Component | 51 | 52 | 53 | 54 |
| Film type | ARF-1(1) | MPF-2(2) | MPF-3(3) | MPF-4(4) |
| PVP-1 | 46 | 46 | 46 | 46 |

(continued)

| Component | Example | | | |
|---|---|---|---|---|
| | 51 | 52 | 53 | 54 |
| Butylene glycol | 6 | 6 | 6 | 6 |
| Talc | 46 | 46 | 46 | 46 |
| Salicylic acid | 2 | 2 | 2 | 2 |
| % solids | 75 | 75 | 75 | 75 |
| Viscosity | 22520 | 22500 | 22500 | 22500 |
| % Particulates after drying | 46 | 46 | 46 | 46 |
| Did coating soak thru to noncoated side? (Y/N) | N | N | N | N |
| (1) AP-3 particle-filled polypropylene microporous film - Amoco Films and Fabrics<br>(2) Exxaire™ particle-filled polyethylene microporous film, 35 GSM - Exxon<br>(3) Particl-filled polyethylene microporous film - Kimberly Clark Corp.<br>(4) Celgard® 2400 polypropylene microporous film - Celgard Inc. | | | | |

Example 55 and Comparative Examples C10 - C12

To demonstrate the effect of the coatings used in the invention on an alternative porous wipe, the same composition and coating technique used in Example 2 was applied to a consolidated nonwoven web formed of polypropylene microfibers. The results in Table 11 below show that at lower percent solids and viscosities the coatings soak through the wipe, but by increasing the viscosity, coatings can be prepared that adequately anchor to the wipe but do not penetrate all the way through to the uncoated side so as to interfere with the intended functionality of the wipe. The consolidated nonwoven web was prepared using apparatus similar to that shown in FIG. 1 of the drawings. Fina 3960, a 350 melt flow index polypropylene resin, was fed into the extruder 13, the temperature of the die 10 was maintained at 371°C, the attenuating air was delivered to the die at a temperature of 390°C. and a flow rate of 5.3 cubic meters per minute. The polypropylene was delivered to the die at a rate of 0.20 kg/hr/cm. The basis weight of the web was 21 grams/m$^2$. The web was then calendered by passing the web, at 15.2 meters/minute, through a nip formed by an upper heated smooth steel roll 24 and a lower unheated 95 Shore A hard rubber roll 25. The nip pressure was 1050 Newtons per lineal centimeter. The temperature of the upper steel roll was 88°C. The caliper of the calendered web was 72 microns.

Table 11

| Component | Consolidated Nonwoven | | | |
|---|---|---|---|---|
| | C10 | C11 | C12 | 55 |
| PVP-2 | 46 | 46 | 46 | 46 |
| Butylene glycol | 6 | 6 | 6 | 6 |
| Talc | 46 | 46 | 46 | 46 |
| Salicylic acid | 2 | 2 | 2 | 2 |
| % Solids | 60 | 65 | 70 | 75 |
| Viscosity | 3500 | 13250 | 37750 | 274000[(1)] |
| % Particulates after drying | 46 | 46 | 46 | 46 |
| Did coating soak thru to noncoated side? (Y/N) | Y | Y | Y | N |
| (1) spindle 4 at 1.5 rpm | | | | |

To demonstrate the effect of filler content on the compositions of the invention, talc was mixed with PVP at various loadings and diluted to 50% solids in isopropyl alcohol. The solutions were then dried and observations made as to the physical nature of the coating. When the percent particulates of the coating compositions exceeds 40% the coating becomes brittle and has a tendency to flake off of the wipe substrate. Results are reported in Table 12 below.

Table 12

| % Talc | % PVP[1] | Comments |
|---|---|---|
| 0 | 100 | Rubbery, tacky |
| 20 | 80 | Rubbery, tacky |
| 40 | 60 | Tough, not tacky |
| 60 | 40 | Brittle, not tacky |
| 80 | 20 | Very brittle, not tacky |
| 100 | 0 | Powdery, not tacky |
| (1) polyvinylpytrolidone - Povidone K-30, 40,000 Mw, BASF Chemical Co. | | |

**Claims**

1. An oil absorbing wipe material suitable for wiping a users skin comprising an oil absorbing porous substrate having two faces wherein the substrate has a transparency of less than 65 percent which porous substrate changes transparency upon absorption of oil, said porous substrate having a generally non-tacky flexible coating on at least a portion of at least one face, said coating comprising a film forming polymer with at least one additional additive which coating is visible on the coated face of the porous substrate and which coating does not penetrate to the opposite face of the porous substrate, wherein the film forming polymer coating penetrates from 10 to 90 percent of the thickness of the oil absorbing porous substrate.

2. The oil absorbing wipe material of claim 1 wherein the coating comprise as least a film forming polymer and a particulate filler wherein the particulate filler comprises 35 to 55 percent by weight of the coating and has an average particle size of from 0.1 to 30 microns.

3. The oil absorbing wipe material of claim 1 or 2 wherein the film forming polymer coating penetrates from 20 to 80 percent of the thickness of the oil absorbing porous substrate.

4. The oil absorbing wipe material of any of claims 1 to 3 wherein the film forming polymer comprises polyvinylpyrrolidone.

5. The oil absorbing wipe material of any of claims 1 to 4 wherein the additional additive is an active or skin modifying agent.

6. The oil absorbing wipe material of claim 5 wherein the active or skin modifying agent is salicylic acid.

7. The oil absorbing wipe material of claim 1 wherein the coating further comprises a gelling agent and/or a filler.

8. The oil absorbing wipe material of any of claims 1 to 7 wherein the porous oil absorbing wipe has a transparency value of about 65% or less when oil free, and which wipe changes transparency by at least 30 percentage points when loaded with about 6 grams or less of oil per square centimeter.

9. The oil absorbing wipe material of claim 8 wherein the wipe, when it has changed transparency, has a transparency of about 90% or greater.

10. The oil absorbing wipe material of claim 8 wherein the wipe changes in transparency by 35% or more when loaded with about 6 grams or less of oil per square centimeter.

11. The oil absorbing wipe material of any of claims 1 to 10 wherein the wipe has a Hand of 8 grams or less.

12. The oil absorbing wipe material of claim 11 wherein the wipe has a Hand of 1 to 6 grams or less.

13. The oil absorbing wipe material of any of claims 1 to 12 wherein the coating is uniformly provided on at least a portion of one face of the oil absorbing wipe material.

14. The oil absorbing wipe material of claim 13 wherein the coating is provided on from 50 to 100 percent of one face of the oil absorbing wipe.

15. The oil absorbing wipe material of claim 14 wherein the coating is continuous or a pattern coating.

16. The oil absorbing wipe material of any of claims 1 to 15 wherein the oil absorbing wipe is a filmlike thermoplastic material, or a consolidated oil absorbing paper wipe.

17. The oil absorbing wipe material of any of claims 1 to 15 wherein the oil absorbing porous substrate comprises a porous stretched film made of a thermoplastic material.

18. The oil absorbing wipe material of claim 17 wherein interstitial volume per unit area of said porous stretched film is in the range of 0.0001-0.005 cm$^3$ as calculated by the following equation:

$$\text{interstitial volume per unit area} = [\text{film thickness (cm)} \times 1 \text{ (cm)} \times \text{void content (\%)}]/100$$

(where the void content is the percentage of voids in the porous film).

19. The oil absorbing wipe material of claim 17 or 18 wherein the porous stretched film comprises a thermoplastic polymer film having from 20 to 60 percent by weight of a filler.

20. The oil absorbing wipe material of claim 19 wherein the porous stretched film contains a non-particulate filler, which is mineral oil.

21. The oil absorbing wipe material of any of claim 17 to 20 wherein the porous stretched film voids have an average size in the range of from 0.2 to 5.0 microns ($\mu$m).

22. The oil absorbing wipe material of any of claims 1 to 15 wherein the porous oil absorbing wipe comprises a consolidated melt-blown web of thermoplastic fibers.

23. The oil absorbing wipe material of claim 22 wherein the thermoplastic fibers are polyolefin microfibers.

24. The oil absorbing wipe material of claim 23 wherein the thermoplastic fibers are polypropylene microfibers.

25. The oil absorbing wipe material of any of claims 22 to 24 wherein the thermoplastic fibers have an average diameter of about 10 micrometers or less, and the wipe has a basis weight of about 40 gm/m$^2$ or less.

26. The oil absorbing wipe material of any of claims 22 to 25 wherein the wipe has a void volume of from 40 to 80 percent.

27. The oil absorbing wipe material of claim 26 wherein the wipe has a void volume of from 50 to 70 percent.

28. The oil absorbing wipe material of any of claims 22 to 27 wherein the average pore size of the wipe material is from 3 to 15 microns.

29. The oil absorbing wipe material of claim 28 wherein the average pore size of the wipe material is from 4 to 8 microns.

30. The oil absorbing wipe material of any of claims 22 to 29 wherein the wipe has an oil absorption capacity of from 0.7 to 6 mg/cm$^2$.

31. The oil absorbing wipe material of any of claims 22 to 30 wherein the wipe has a basis weight of from 10 to 30 gm/m$^2$.

32. A method for forming a flexible coating on an oil absorbing wipe material suitable for wiping a user's skin comprising providing an oil absorbing porous substrate having two faces wherein the substrate has a transparency of less than 65 percent which porous substrate changes transparency upon absorption of oil, coating the porous substrate on at least a portion of at least one face with a coating solution comprising at least a film forming polymer, a particulate filler and an evaporative solvent with at least one additional additive, the coating solution having a viscosity of from 2000 to 100,000 cps and a percent solids of 60 to 80 percent wherein the coating is visible on the coated face of the porous substrate and which coating does not penetrate to the opposite face of the porous substrate.

33. The method of forming an oil absorbing wipe material of claim 32 wherein the oil absorbing wipe is a consolidated oil absorbing paper wipe and the coating solution has a viscosity of from 10,000 to 100,000 cps.

34. The method of forming an oil absorbing wipe material of claim 32 or 33 wherein the coating comprises at least a film forming polymer and a particulate filler such that the dried coating has 35 to 55 percent particulate filler to other nonparticulate solids said filler having an average particle size of from 0.1 to 30 microns.

35. The method of forming an oil absorbing wipe material of claim 34 wherein the particulate filler comprises 40 to 50 percent by weight of the solids.

36. The method of forming an oil absorbing wipe material of any of claims 32 to 35 wherein the film forming polymer coating penetrates from 10 to 90 percent of the thickness of the oil absorbing porous substrate.

**Patentansprüche**

1. Öl absorbierendes Wischtuchmaterial, das zum Abwischen der Haut eines Benutzers geeignet ist, umfassend ein Öl absorbierendes poröses Substrat mit zwei Seiten, wobei das Substrat eine Transparenz von weniger als 65 Prozent aufweist, wobei das poröse Substrat bei Absorption von Öl seine Transparenz ändert, das poröse Substrat eine im allgemeinen nichtklebrige flexible Beschichtung auf mindestens einem Teil mindestens einer Seite aufweist, wobei die Beschichtung ein filmbildendes Polymer mit mindestens einem zusätzlichen Additiv umfasst, wobei die Beschichtung auf der beschichteten Seite des porösen Substrats sichtbar ist und die Beschichtung nicht zur gegenüberliegenden Seite des porösen Substrats penetriert, wobei die Beschichtung aus filmbildendem Polymer 10 bis 90 Prozent der Dicke des Öl absorbierenden porösen Substrats penetriert.

2. Öl absorbierendes Wischtuchmaterial nach Anspruch 1, wobei die Beschichtung mindestens ein filmbildendes Polymer und einen teilchenförmigen Füllstoff umfasst, wobei der teilchenförmige Füllstoff 35 bis 55 Gewichtsprozent der Beschichtung ausmacht und eine durchschnittliche Teilchengröße von 0,1 bis 30 Mikron aufweist.

3. Öl absorbierendes Wischtuchmaterial nach Anspruch 1 oder 2, wobei die Beschichtung aus filmbildendem Polymer 20 bis 80 Prozent der Dicke des Öl absorbierenden porösen Substrats penetriert.

4. Öl absorbierendes Wischtuchmaterial nach einem der Ansprüche 1 bis 3, wobei das filmbildende Polymer Polyvinylpyrrolidon umfasst.

5. Öl absorbierendes Wischtuchmaterial nach einem der Ansprüche 1 bis 4, wobei das zusätzliche Additiv ein aktives oder hautmodifizierendes Mittel ist.

6. Öl absorbierendes Wischtuchmaterial nach Anspruch 5, wobei das aktive oder hautmodifizierende Mittel Salicylsäure ist.

7. Öl absorbierendes Wischtuchmaterial nach Anspruch 1, wobei die Beschichtung ferner ein Geliermittel und/oder einen Füllstoff umfasst.

8. Öl absorbierendes Wischtuchmaterial nach einem der Ansprüche 1 bis 7, wobei das poröse Öl absorbierende Wischtuch einen Transparenzwert von etwa 65% oder weniger aufweist, wenn es ölfrei ist, und wobei das Wischtuch seine Transparenz bei Beladung mit etwa 6 Gramm oder weniger Öl pro Quadratzentimeter um mindestens 30 Prozentpunkte ändert.

9. Öl absorbierendes Wischtuchmaterial nach Anspruch 8, wobei das Wischtuch, wenn es seine Transparenz geändert hat, eine Transparenz von etwa 90% oder mehr aufweist.

10. Öl absorbierendes Wischtuchmaterial nach Anspruch 8, wobei das Wischtuch seine Transparenz bei Beladung mit etwa 6 Gramm oder weniger Öl pro Quadratzentimeter um 35% oder mehr ändert.

11. Öl absorbierendes Wischtuchmaterial nach einem der Ansprüche 1 bis 10, wobei das Wischtuch eine Griffigkeit von 8 Gramm oder weniger aufweist.

12. Öl absorbierendes Wischtuchmaterial nach Anspruch 11, wobei das Wischtuch eine Griffigkeit von 1 bis 6 Gramm oder weniger aufweist.

13. Öl absorbierendes Wischtuchmaterial nach einem der Ansprüche 1 bis 12, wobei die Beschichtung einheitlich auf mindestens einem Teil einer Seite des Öl absorbierenden Wischtuchmaterials bereitgestellt ist.

14. Öl absorbierendes Wischtuchmaterial nach Anspruch 13, wobei die Beschichtung auf 50 bis 100 Prozent einer Seite des Öl absorbierenden Wischtuchs bereitgestellt ist.

15. Öl absorbierendes Wischtuchmaterial nach Anspruch 14, wobei die Beschichtung kontinuierlich oder eine gemusterte Beschichtung ist.

16. Öl absorbierendes Wischtuchmaterial nach einem der Ansprüche 1 bis 15, wobei das Öl absorbierende Wischtuch ein folienartiges thermoplastisches Material oder ein konsolidiertes Öl absorbierendes Papierwischtuch ist.

17. Öl absorbierendes Wischtuchmaterial nach einem der Ansprüche 1 bis 15, wobei das Öl absorbierende poröse Substrat eine poröse gestreckte Folie aus einem thermoplastischen Material umfasst.

18. Öl absorbierendes Wischtuchmaterial nach Anspruch 17, wobei das Zwischenraumvolumen pro Flächeneinheit der porösen gestreckten Folie im Bereich von 0,0001-0,005 cm$^3$ liegt, wie anhand der folgenden Gleichung berechnet:

$$\text{Zwischenraumvolumen pro Flächeneinheit} = [\text{Folien-dicke (cm) x 1 (cm) x Hohlraumgehalt (\%)}]/100$$

(wobei der Hohlraumgehalt der Prozentanteil an Hohlräumen in der porösen Folie ist).

19. Öl absorbierendes Wischtuchmaterial nach Anspruch 17 oder 18, wobei die poröse gestreckte Folie eine Folie aus thermoplastischem Polymer mit 20 bis 60 Gewichtsprozent eines Füllstoffs umfaßt.

20. Öl absorbierendes Wischtuchmaterial nach Anspruch 19, wobei die poröse gestreckte Folie einen nicht teilchenförmigen Füllstoff enthält, der ein Mineralöl ist.

21. Öl absorbierendes Wischtuchmaterial nach einem der Ansprüche 17 bis 20, wobei die Hohlräume der porösen gestreckten Folie eine durchschnittliche Größe im Bereich von 0,2 bis 5,0 Mikron ($\mu$m) aufweisen.

22. Öl absorbierendes Wischtuchmaterial nach einem der Ansprüche 1 bis 15, wobei das poröse Öl absorbierende Wischtuch eine konsolidierte schmelzgeblasene Bahn von thermoplastischen Fasern umfasst.

23. Öl absorbierendes Wischtuchmaterial nach Anspruch 22, wobei die thermoplastischen Fasern Polyolefin-Mikrofasern sind.

24. Öl absorbierendes Wischtuchmaterial nach Anspruch 23, wobei die thermoplastischen Fasern Polypropylen-Mikrofasern sind.

25. Öl absorbierendes Wischtuchmaterial nach einem der Ansprüche 22 bis 24, wobei die thermoplastischen Fasern einen durchschnittlichen Durchmesser von etwa 10 Mikrometer oder weniger aufweisen und das Wischtuch ein Flächengewicht von etwa 40 g/m$^2$ oder weniger aufweist.

26. Öl absorbierendes Wischtuchmaterial nach einem der Ansprüche 22 bis 25, wobei das Wischtuch ein Hohlraumvolumen von 40 bis 80 Prozent aufweist.

**27.** Öl absorbierendes Wischtuchmaterial nach Anspruch 26, wobei das Wischtuch ein Hohlraumvolumen von 50 bis 70 Prozent aufweist.

**28.** Öl absorbierendes Wischtuchmaterial nach einem der Ansprüche 22 bis 27, wobei die durchschnittliche Porengröße des Wischtuchmaterials 3 bis 15 Mikron beträgt.

**29.** Öl absorbierendes Wischtuchmaterial nach Anspruch 28, wobei die durchschnittliche Porengröße des Wischtuchmaterials 4 bis 8 Mikron beträgt.

**30.** Öl absorbierendes Wischtuchmaterial nach einem der Ansprüche 22 bis 29, wobei das Wischtuch eine Ölabsorptionskapazität von 0,7 bis 6 mg/cm$^2$ aufweist.

**31.** Öl absorbierendes Wischtuchmaterial nach einem der Ansprüche 22 bis 30, wobei das Wischtuch ein Flächengewicht von 10 bis 30 g/m$^2$ aufweist.

**32.** Verfahren zum Bilden einer flexiblen Beschichtung auf einem Öl absorbierenden Wischtuchmaterial, das zum Abwischen der Haut eines Benutzers geeignet ist, umfassend das Bereitstellen eines Öl absorbierenden porösen Substrats mit zwei Seiten, wobei das Substrat eine Transparenz von weniger als 65 Prozent aufweist und bei Absorption von Öl seine Transparenz ändert, Beschichten des porösen Substrats auf mindestens einem Teil mindestens einer Seite mit einer Beschichtungslösung, die mindestens ein filmbildendes Polymer, einen teilchenförmigen Füllstoff und ein verdampfendes Lösungsmittel mit mindestens einem zusätzlichen Additiv umfasst, wobei die Beschichtungslösung eine Viskosität von 2000 bis 100.000 cPs und einen prozentualen Feststoffgehalt von 60 bis 80 Prozent aufweist, wobei die Beschichtung auf der beschichteten Seite des porösen Substrats sichtbar ist und die Beschichtung nicht zur gegenüberliegenden Seite des porösen Substrats penetriert.

**33.** Verfahren zum Bilden eines Öl absorbierenden Wischtuchmaterials nach Anspruch 32, wobei das Öl absorbierende Wischtuch ein konsolidiertes Ö1 absorbierendes Papierwischtuch ist und die Beschichtungslösung eine Viskosität von 10.000 bis 100.000 cPs aufweist.

**34.** Verfahren zum Bilden eines Öl absorbierenden Wischtuchmaterials nach Anspruch 32 oder 33, wobei die Beschichtung mindestens ein filmbildendes Polymer und einen teilchenförmigen Füllstoff umfasst, so dass die getrocknete Beschichtung 35 bis 55 Prozent teilchenförmigen Füllstoff, bezogen auf andere nicht teilchenförmige Feststoffe, aufweist, wobei der Füllstoff eine durchschnittliche Teilchengröße von 0,1 bis 30 Mikron aufweist.

**35.** Verfahren zum Bilden eines Öl absorbierenden Wischtuchmaterials nach Anspruch 34, wobei der teilchenförmige Füllstoff 40 bis 50 Gewichtsprozent der Feststoffe ausmacht.

**36.** Verfahren zum Bilden eines Öl absorbierenden Wischtuchmaterials nach einem der Ansprüche 32 bis 35, wobei die Beschichtung aus filmbildendem Polymer 10 bis 90 Prozent der Dicke des Öl absorbierenden porösen Substrats penetriert.

**Revendications**

**1.** Matériau à lingettes d'absorption d'huile approprié pour essuyer la peau d'un utilisateur, qui comprend un substrat poreux d'absorption d'huile qui possède deux faces, dans lequel le substrat possède une transparence inférieure à 65 %, lequel substrat poreux voit sa transparence se modifier lors de l'absorption d'huile, ledit substrat poreux possédant un revêtement souple généralement non collant sur au moins une partie d'au moins une face, ledit revêtement comprenant un polymère filmogène avec au moins un additif supplémentaire, lequel revêtement est visible sur la face enduite du substrat poreux et lequel revêtement ne pénètre pas jusqu'à la face opposée du substrat poreux, dans lequel le revêtement à base du polymère filmogène traverse jusqu'à 10 à 90% de l'épaisseur du substrat poreux d'absorption d'huile.

**2.** Matériau à lingettes d'absorption d'huile selon la revendication 1, dans lequel le revêtement comprend au moins un polymère filmogène et une charge particulaire, dans lequel la charge particulaire constitue de 35 à 55 % en poids du revêtement et possède une taille moyenne de particules de 0,1 à 30 microns.

**3.** Matériau à lingettes d'absorption d'huile selon la revendication 1 ou 2, dans lequel le revêtement à base du polymère filmogène traverse jusqu'à 20 à 80 % de l'épaisseur du substrat poreux d'absorption d'huile.

4. Matériau à lingettes d'absorption d'huile selon l'une quelconque des revendications 1 à 3, dans lequel le polymère filmogène comprend du polyvinylpyrrolidone.

5. Matériau à lingettes d'absorption d'huile selon l'une quelconque des revendications 1 à 4, dans lequel l'additif supplémentaire est un agent actif ou de modification de la peau.

6. Matériau à lingettes d'absorption d'huile selon la revendication 5, dans lequel l'agent actif ou de modification de la peau est de l'acide salicylique.

7. Matériau à lingettes d'absorption d'huile selon la revendication 1, dans lequel le revêtement comprend en outre un agent de gélification et/ou une charge.

8. Matériau à lingettes d'absorption d'huile selon l'une quelconque des revendications 1 à 7, dans lequel la lingette poreuse d'absorption d'huile possède une valeur de transparence d'environ 65% ou moins en l'absence d'huile, et dont la lingette voit se modifier sa transparence par au moins 30 points de pourcentage lorsqu'il est chargé avec environ 6 g ou moins d'huile par centimètre carré.

9. Matériau à lingettes d'absorption d'huile selon la revendication 8, dans lequel la lingette, lorsque sa transparence a été modifiée, possède une transparence d'environ 90% ou plus.

10. Matériau à lingettes d'absorption de sébum selon la revendication 8, dans lequel la lingette voit se modifier sa transparence par 35% ou plus lorsqu'il est chargé avec environ 6 g ou moins d'huile par centimètre carré.

11. Matériau à lingettes d'absorption d'huile selon l'une quelconque des revendications 1 à 10, dans lequel la lingette possède une main de 8 g ou moins.

12. Matériau à lingettes d'absorption d'huile selon la revendication 11, dans lequel la lingette possède une main de 1 à 6 g ou moins.

13. Matériau à lingettes d'absorption d'huile selon l'une quelconque des revendications 1 à 12, dans lequel le revêtement est uniformément appliqué sur au moins une partie d'une face du matériau à lingettes d'absorption d'huile.

14. Matériau à lingettes d'absorption d'huile selon la revendication 13, dans lequel le revêtement est appliqué sur 50 à 100 % d'une face de la lingette d'absorption d'huile.

15. Matériau à lingettes d'absorption d'huile selon la revendication 14, dans lequel le revêtement est continu ou est un revêtement à motifs.

16. Matériau à lingettes d'absorption d'huile selon l'une quelconque des revendications 1 à 15, dans lequel la lingette d'absorption d'huile est un matériau thermoplastique de type film ou une lingette en papier consolidé d'absorption d'huile.

17. Matériau à lingettes d'absorption d'huile selon l'une quelconque des revendications 1 à 15, dans lequel le substrat poreux d'absorption d'huile comprend un film étiré poreux fabriqué à partir d'un matériau thermoplastique.

18. Matériau à lingettes d'absorption d'huile selon la revendication 17, dans lequel le volume interstitiel par unité de surface dudit film étiré poreux se situe dans la plage de 0,0001 à 0,005 cm$^3$ tel que calculé à l'aide de l'équation suivante :

```
Volume interstitiel par unité de surface = [épaisseur
du film (cm) x 1 (cm) x teneur en vide (%)]/100
```

(où la teneur en vide est le pourcentage de vides dans le film poreux).

**19.** Matériau à lingettes d'absorption d'huile selon la revendication 17 ou 18, dans lequel le film étiré poreux comprend un film polymère thermoplastique qui contient de 20 à 60 % en poids d'une charge.

**20.** Matériau à lingettes d'absorption d'huile selon la revendication 19, dans lequel le film étiré poreux contient une charge non particulaire, laquelle est une huile minérale.

**21.** Matériau à lingettes d'absorption d'huile selon l'une quelconque des revendications 17 à 20, dans lequel les vides du film étiré poreux possèdent une taille moyenne située dans la plage de 0,2 à 5,0 microns ($\mu$m).

**22.** Matériau à lingettes d'absorption d'huile selon l'une quelconque des revendications 1 à 15, dans lequel la lingette poreuse d'absorption d'huile comprend un tissu fondu-soufflé consolidé composé de fibres thermoplastiques.

**23.** Matériau à lingettes d'absorption d'huile selon la revendication 22, dans lequel les fibres thermoplastiques sont des microfibres de polyoléfines.

**24.** Matériau à lingettes d'absorption d'huile selon la revendication 23, dans lequel les fibres thermoplastiques sont des microfibres de polypropylène.

**25.** Matériau à lingettes d'absorption d'huile selon l'une quelconque des revendications 22 à 24, dans lequel les fibres thermoplastiques ont un diamètre moyen d'environ dix micromètres ou moins et la lingette possède une masse surfacique d'environ 40 g/m$^2$ ou moins.

**26.** Matériau à lingettes d'absorption d'huile selon l'une quelconque des revendications 22 à 25, dans lequel la lingette possède un volume de vide de 40 à 80 %.

**27.** Matériau à lingettes d'absorption d'huile selon la revendication 26, dans lequel la lingette possède un volume de vide de 50 à 70 %.

**28.** Matériau à lingettes d'absorption d'huile selon l'une quelconque des revendications 22 à 27, dans lequel la taille moyenne des pores du matériau à lingettes varie de 3 à 15 microns.

**29.** Matériau à lingettes d'absorption d'huile selon la revendication 28, dans lequel la taille moyenne des pores du matériau à lingettes varie de 4 à 8 microns.

**30.** Matériau à lingettes d'absorption d'huile selon l'une quelconque des revendications 22 à 29, dans lequel la lingette possède une capacité d'absorption d'huile de 0,7 à 6 mg/cm$^2$.

**31.** Matériau à lingettes d'absorption d'huile selon l'une quelconque des revendications 22 à 30, dans lequel la lingette possède une masse surfacique de 10 à 30 g/m$^2$.

**32.** Procédé de formation d'un revêtement souple sur un matériau à lingettes d'absorption d'huile approprié pour essuyer la peau d'un utilisateur, qui comprend la fourniture d'un substrat poreux d'absorption d'huile qui possède deux faces, dans lequel le substrat possède une transparence inférieure à 65 %, lequel substrat poreux voit se modifier sa transparence lors de l'absorption d'huile, le revêtement du substrat poreux sur au moins une partie d'au moins une face avec une solution de revêtement qui comprend au moins un polymère filmogène, une charge particulaire et un solvant d'évaporation avec au moins un additif supplémentaire, la solution de revêtement ayant une viscosité de 2000 à 100 000 cps et un pourcentage de solides de 60 à 80 %, dans lequel le revêtement est visible sur la face enduite du substrat poreux et lequel revêtement ne pénètre pas jusqu'à la face opposée du substrat poreux.

**33.** Procédé de formation d'un matériau à lingettes d'absorption d'huile selon la revendication 32, dans lequel la lingette d'absorption d'huile est une lingette en papier consolidé d'absorption d'huile et la solution de revêtement possède une viscosité de 10 000 à 100 000 cps.

**34.** Procédé de formation d'un matériau à lingettes d'absorption d'huile selon la revendication 32 ou 33, dans lequel le revêtement comprend au moins un polymère filmogène et d'une charge particulaire de sorte que le revêtement séché contient de 35 à 55 % de charge particulaire par rapport aux autres solides non particulaires, ladite charge ayant un taille moyenne de particules qui varie de 0,1 à 30 microns.

**35.** Procédé de formation d'un matériau à lingettes d'absorption d'huile selon la revendication 34, dans lequel la charge particulaire est composée de 40 à 50 % en poids de solides.

**36.** Procédé de formation d'un matériau à lingettes d'absorption d'huile selon l'une quelconque des revendications 32 à 35, dans lequel le revêtement à base du polymère filmogène traverse jusqu'à 10 à 90 % de l'épaisseur du substrat poreux d'absorption d'huile.

*Fig. 1*

Fig. 2

Fig. 3

**Fig. 4**

**Fig. 5**

**Fig. 6**

**Fig. 7**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 4045591 A
- JP 6319664 A
- JP 56008606 A
- US 4643939 A
- JP 5018392 A
- JP 9335451 A
- WO 9929220 A
- US 4532937 A
- US 6214362 B

- US 4818463 A
- US 3825379 A
- US 4907174 A
- US 4986743 A
- US 3971373 A
- US 4755178 A
- US 5830487 A
- US 4569343 A
- US 5744149 A

**Non-patent literature cited in the description**

- **Wente Van A.** Superfine Thermoplastic Fibers. *Industrial Engineering Chemistry,* 1956, vol. 48, 1342

- **Wente, V.A. ; Boone, C. D. ; Fluharty, E.L.** Manufacture of Superfine Organic Fibers. Naval Research Laboratories, 25 May 1954